# EUROPEAN PATENT APPLICATION

(11) **EP 2 738 261 A2**
(43) Date of publication of application: **04.06.2014**
(21) Application number: 13195044.6
(22) Date of filing: 29.11.2013
(51) Int. Cl.: C12P 7/44, C08L 67/02

(54) **Process for the enzymatic production of terephthalic acid**

(30) Priority: 30.11.2012 KR 20120138374
(71) Applicant: Samsung Electronics Co., Ltd, Gyeonggi-do 443-742 (KR)
(72) Inventor: Choi, Won Jae, Gyeonggi-do (KR); Ahn, Jin Ho, Gyeonggi-do (KR); Byun, Jong Won, Gyeonggi-do (KR); Ha, Young Wan, Seoul (KR)
(74) Representative: Grünecker, Kinkeldey, Stockmair & Schwanhäusser

(57) **Abstract**

A method of biologically producing an terephthalic acid or a derivative thereof by contacting a substrate containing an aromatic carboxylic acid with a biocatalyst that adds a carboxyl group at the para-position of the aromatic carboxylic acid.

## Description

### BACKGROUND OF THE INVENTION

### Field of the Invention

The present invention relates to the biological production of terephthalic acid and its derivatives. More particularly, the present invention relates to a process of biologically producing terephthalic acid and its derivatives by degrading lignin through chemical or biological conversion and biologically converting the lignin breakdown product into terephthalic acid or derivatives thereof.

### Description of the Related Art

A terephthalic acid is a useful starting material for a wide range of applications, such as a polyester fiber of polyethylene terephthalate (PET) and polytrimethylene terephthalate (PTT), a packing material and bottle material. Particularly, PET has been widely used as a packing material due to its transparence, mechanical strength, and gas barrier capacity. The terephthalic acid as a raw material preparing for PET is prepared at an amount of 50,000,000 tons every year, and 100,000 tons to 800,000 tons in a single plant every year.

PET is synthetic polymer consisting of terephthalic acid and mono ethylene glycol, both derived from petroleum. Specifically terephthalic acid is industrially manufactured from p-xylene via catalytic oxidation. Unexpected increases in crude oil prices results in high manufacturing costs for aromatic raw materials such as benzene, toluene, and xylene, indicating that terephthalic acid production is very much dependent on crude oil price. Furthermore, conventional petroleum-based terephthalic acid production is recognized as nonrenewable and generates a lot of greenhouse gases. In this context, there is an increasing demand for alternative route for terephthalic acid production via industrial biotechnology using renewable biomass as feedstock.

Terephthalic acid is prepared by gaseous oxidation from para-xylene. More specifically, crude terephthalic acid is prepared by the oxidation with aerating the para-xylene in acetic acid solvent in the presence of catalyst such as cobalt, manganese or bromide, and is dissolved in water and reduced by hydrogen to produce the purified terephthalic acid. This method is referred to as the Amoco MC method.

However, the process of chemically synthesizing terephthalic acid has various serious problems, namely dangerous working conditions requiring high temperature and pressure and environmental contamination caused by the complicated separation and purification steps due to by-products and chemical wastes produced in the separation and purification steps.

Bromide is used as a reaction accelerating agent. Although the bromide functions as the initiation and acceleration of oxidation, it causes device corrosion, and therefore, results in a periodic need to replace the production facilities. Furthermore, bromide is very toxic to human body. When a very small amount of bromide is inhaled or contacted, it is very lethal to humans, worsens the working environment, and causes air contamination.

Compared with other conventional reactions, the Amoco MC method uses an acetic acid as a reaction solvent. Acetic acid helps the catalytic action of the reaction by increasing the complex formation between a heavy metal catalyst and a reaction accelerating agent, but has some disadvantages. That is, when acetic acid is used at a high temperature, it needs an expensive anticorrosive material used in the reactor and its surrounding equipment due to corrosive capacity, thereby increasing the production cost. Like bromide, acetic acid is very harmful to humans.

Accordingly, some methods for an environmentally-friendly, simple, and cost effective preparation of terephthalic acid have been developed, including a process of biologically preparing terephthalic acid from petroleum-based feedstock.

Among the biological process using fossil fuel, terephthalic acid has been prepared from hexadecane and p-xylene by using Nocardia culture (SU419509). Both methyl groups of p-xylene were oxidized simultaneously by using Burkholderia culture (US6461840B1). However, these methods do not represent truely environmentally-friendly biological processes, because they still use fossil fuel as a substrate.

US2011/0207185A1 discloses the preparation of terephthalic acid by using a recombinant microorganism having a (2-hydroxy-3-methyl-4-oxobutoxy)phosphonate, p-toluate, or terephthalate pathway. However, the method requires glucose as a substrate, which is mainly derived from edible resources.

There is therefore impertinent strong need for a biological process of preparing terephthalic acid from a renewable biomass.

### SUMMARY OF THE INVENTION

An embodiment of the present invention provides a biological process of producing terephthalic acid or its derivative, which is of higher specificity, compared to chemical processes.

Another embodiment provides a biological process of producing a terephthalic acid and its derivative in an environment-friendly manner from a lignin degradation product which is degraded economically and effectively from lignin.

A further embodiment pertains to a biological process of producing terephthalic acid or a derivative thereof. More particularly, terephthalic acid or a derivative thereof can be biologically prepared from an aromatic carboxylic acid or a derivative thereof.

Still further embodiment provides the production of terephthalic acid or a derivative thereof from lignin of a recyclable biomass. In this regard, lignin is chemically and/or biologically degraded to give lignin breakdown products including aromatic monomers, for example benzoic acid followed by biological conversion of the breakdown products into terephthalic acid or a derivative thereof.

Focusing on the synthesis of terephthalic acid or a derivative thereof from non-edible biomass, the present invention is configured to chemically or biologically degrade lignin, an inexpensive raw material, into aromatic monomers and to chemically or biologically convert the aromatic monomers into terephthalic acid, thus eliminating dependence on petrochemical materials.

By using a biological process in which a substrate including an aromatic carboxylic acid having a p-hydroxyl group, or a derivative thereof is contacted with a biocatalyst having an activity to introducing p-carboxyl group, an embodiment of the present invention allows terephthalic acid or a derivative thereof to be produced in an environment-friendly manner and at higher specificity, compared to a chemical process.

An embodiment provides the production of terephthalic acid or a derivative thereof from lignin. In this regard, lignin may be degraded to give lignin breakdown products including aromatic monomers, followed by biological conversion of the breakdown products into terephthalic acid or a derivative thereof.

### BREIF DESCRIPTION OF THE DRAWINGS

The above and other objects, features and other advantages of the present invention will be more clearly understood from the following detailed description taken in conjunction with the accompanying drawings, in which:
FIG. 1 shows HPLC chromatograms of a standard solution of enzyme reaction intermediates comprising p-hydroxybenzoic acid, benzoic acid and terephthalic acid, as quantitatively measured by Waters e2695 HPLC and Waters 2489 UV/VIS (253 nm, 280 nm) detector.
FIGS. 2A and 2B shows mass spectra of the produced terephthalic acid obtained by the enzyme reaction (FIG. 2A) and the standard solution of terephthalic acid (FIG. 2B).

### DESCRIPTION OF THE PREFERRED EMBODIMENTS

An embodiment of the present invention addresses a process of biologically producing an aromatic carboxylic acid represented by the following Chemical Formula 1 or a derivative thereof, including contacting a substrate including an aromatic carboxylic acid represented by the following Chemical Formula 2, with a biocatalyst having a catalytic activity to introducing a carboxyl group at p-position, as illustrated in the following Reaction Scheme 1. 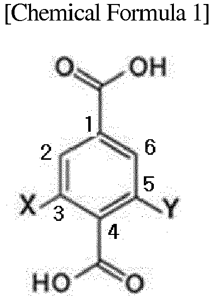 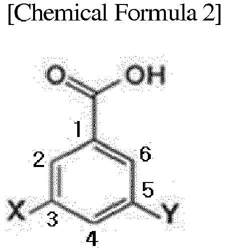 wherein, X and Y, which may be the same or different, are independently hydrogen, hydroxy, or C1-C4 alkoxy. The C1-C4 alkoxy may be linear or branched, and is preferably methoxy or ethoxy.

The exemplified aromatic carboxylic acid represented by the following Chemical Formula 1 or an derivative thereof are a terephthalic acid, m-hydroxy terephthalic acid, 3,5-dihydroxy terephthalic acid, 3-methoxy terephthalic acid, 3,5-dimethoxy terephthalic acid, and the like.

The compound of Chemical Formula 2 corresponds to the compound of Chemical Formula 1 and can be obtained by the petrochemical method or biological method. For example, the compound of Chemical Formula 2 may be obtained by the industrial chemical process including a partial oxidation of toluene or the biological method using a microorganism, such as *S. maritimus* and the like (Shuhei et. al., Microbial Cell Factories 2012, 11:49). Alternatively, the compound of Chemical Formula 2 can be obtained from the compound of Chemical Formula 3. The examples of the compound represented by Chemical Formula 2 include benzoic acid, m-hydroxybenzoic acid, 3,5-dihydroxybenzoic acid, 3-methoxybenzoic acid, 3,5-dimethoxybenzoic acid, and the like.

The aromatic carboxylic acid represented by Chemical Formula 2 may be prepared by contacting the substrate including the aromatic carboxylic acid represented by Chemical Formula 3, with a biocatalyst having an activity to remove the p-hydroxy group of Chemical Formula 3. More specifically, before or simultaneously contacting with the biocatalyst having an activity to introduce the carboxyl group to p-position, the method of producing the compound represented by Chemical Formula 1 further includes a step of producing an aromatic carboxylic acid represented by Chemical Formula 2 which is prepared by contacting the substrate including the aromatic carboxylic acid represented by Chemical Formula 3, with a biocatalyst having an activity to remove the p-hydroxyl group of Chemical Formula 3. 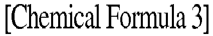 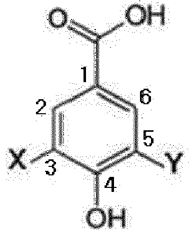 wherein, X and Y, which may be the same or different, are independently hydrogen, hydroxyl, or C1-C4 alkoxy. The C1-C4 alkoxy may be linear or branched, with preference for methoxy or ethoxy.

The compound of Chemical Formula 3 which corresponds to the compound of Chemical Formula 2, may be produced by using a petrochemical process or a biological process. For example, it may be produced using a commercial chemical process based on the Kolbe-Schmitt reaction, or through an aromatic amino acid biosynthesis pathway (Edwin Ritzer and Rudolf Sundermann "Hydroxycarboxylic Acids, Aromatic" in Ullmann's Encyclopedia of Industrial Chemistry 2002, Wiley-VCH, Weinheim; Biotechnol Bioeng. 2001 Dec;76(4):376-90). Alternatively, the compound of Chemical Formula 3 may be obtained from a breakdown product of lignin after chemical and/or biological degradation, or from a compound of Chemical Formula 4, as will be further explained. The degradation of lignin may be carried out using at least one selected from the group consisting of pyrolysis, gasification, hydrogenolysis, acidolysis, alkaline lysis, chemical oxidation, hydrolysis under supercritical conditions, and enzymolysis. Preferred examples of the compound of Chemical Formula 3 include 4-hydroxybenzoic acid, 3,4-dihydroxybenzoic acid, 4,5-dihydroxybenzoic acid, 3,4,5-trihydroxybenzoic acid, 3-methoxy-4-hydroxybenzoic acid, 4-hydroxy-5-methoxybenzoic acid, and 3,5-dimethoxy-4-hydroxybenzoic acid.

In one embodiment, the aromatic carboxylic acid having a p-hydroxy group of Chemical Formula 3 may be prepared from an aromatic aldehyde having a p-hydroxyl group of Chemical Formula 4 by chemical oxidation or biocatalytic oxidation. 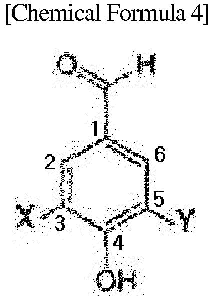 wherein, X and Y, which may be the same or different, are independently hydrogen, hydroxy, or C1-C4 alkoxy. The C1-C4 alkoxy may be linear or branched, with preference for methoxy or ethoxy. Examples of the compound of Chemical Formula 4 are p-hydroxybenzaldehyde, vanillin, and syringaldehyde.

The compound of Chemical Formula 4, which corresponds to the compound of Chemical Formula 3, may be produced using a petrochemical process or a biological process. For example, it may be obtained by chemically or biologically degrading lignin. In one embodiment, the process of the present invention may further include degrading lignin to provide a breakdown product of lignin including the aromatic aldehyde having a p-hydroxyl group of Chemical Formula 4; and oxidizing the breakdown product of lignin to convert the aromatic aldehyde having a p-hydroxy group into an aromatic carboxylic acid having p-hydroxyl group of Chemical Formula 3.

A process of producing a terephthalic acid or a derivative thereof from an aromatic aldehyde in accordance with an embodiment of the present invention is illustrated in Reaction Scheme 1. Specifically, the aromatic aldehyde having a p-hydroxy group of Chemical Formula 4 is chemically or biologically oxidized into an aromatic carboxylic acid having a p-hydroxy group of Chemical Formula 3, followed by biological removal of the p-hydroxy group to give a p-hydroxy-free aromatic carboxylic acid represented by Chemical Formula 2, and then introducing a carboxylic acid at p-position to produce a terephthalic acid or a derivative thereof represented by Chemical Formula 1.

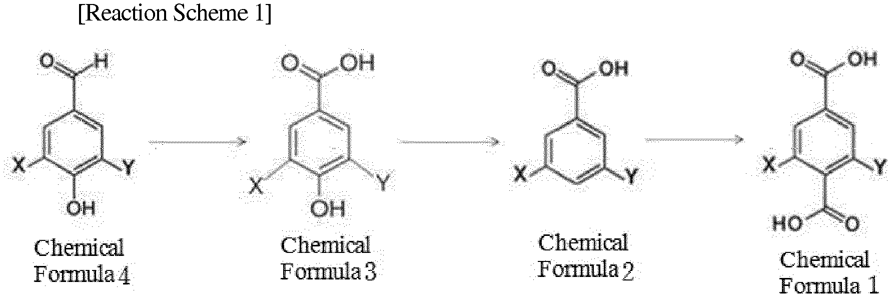

When X and Y in the compounds of Chemical Formulae 1 to 4 are not simultaneously a hydrogen substituent, the process of the present invention may further comprise removing at least one substituent selected from the group consisting of hydroxy and C1-C4 alkoxy at either or both positions 3' and 5' of the benzene ring. The removal of the substituent may be carried out prior to, simultaneously with, or after each step of Reaction Scheme 1.

According to one embodiment, when X and Y are not simultaneously hydrogen in Chemical Formula 2, the process of the present invention may further comprises contacting the substrate with a biocatalyst having the catalytic activity to remove a substituent selected from the group consisting of hydroxy and C1-C4 alkoxy at either or both of positions 3 and 5 on the benzene ring before, simultaneously with, or after contacting with the biocatalyst having the catalytic activity to introducing a carboxyl group at p-position. For example, when X and Y in Chemical Formula 2 are hydrogen and methoxy, respectively, the introduction of carboxyl group at p-position and the removal of methoxy of Y may give a terephthalic acid.

According to one embodiment, when X and Y are not simultaneously hydrogen in Chemical Formula 3, the process of the present invention may further include contacting the substrate with a biocatalyst having the catalytic activity to remove a substituent selected from the group consisting of hydroxyl group and C1-C4 alkoxy at either or both of positions 3 and 5 on the benzene ring before, simultaneously with, or after contacting with the biocatalyst having the catalytic activity to remove a hydroxyl group at p-position.

According to another embodiment, when X and Y are not simultaneously hydrogen in Chemical Formula 4, the process of the present invention may further include contacting the substrate with a biocatalyst having the catalytic activity to remove a substituent selected from the group consisting of hydroxy and C1-C4 alkoxy at either or both of positions 3 and 5 on the benzene ring before, simultaneously with, or after the oxidation.

The process illustrated in Reaction Scheme 1, involves a biocatalyst having a catalytic activity to introduce p-carboxyl group to the aromatic carboxylic acid by contacting with a substrate including an aromatic carboxylic acid of Chemical Formula 2, a biocatalyst having a catalytic activity to remove p-hydroxy from an aromatic carboxylic acid having a p-hydroxy group of Chemical Formula 3, and a biocatalyst having a catalytic activity to remove at least one substituent selected from the group consisting of hydroxy and C1-C4 alkoxy at either or both 3-and 5-positions on the aromatic carboxylic acid.

Any enzyme may be used in the present invention as a biocatalyst having a catalytic activity to introduce p-carboxyl group to the aromatic carboxylic acid, so long as it can introduce a carboxylic group at p-position of the benzene ring from the aromatic carboxylic acid or its derivatives. The examples of biocatalyst having a catalytic activity to introduce p-carboxyl group to the aromatic carboxylic acid include at least one selected from the group consisting of aminobenzoate decarboxylase (EC 4.1.1.24), 3-Hydroxy-2-methylpyridine-4,5-dicarboxylate 4-decarboxylase (EC 4.1.1.51), 4,5-Dihydroxyphthalate decarboxylase (EC 4.1.1.55), Orsellinate decarboxylase (EC 4.1.1.58) (Eur. J. Biochem, 1971, 26:22(4);485-8), Gallate decarboxylase (EC 4.1.1.59) (J.Bacteriol. 1987, 169(5):1886-90), 4-hydroxybenzoate decarboxylase (EC 4.1.1.61), Protocatechuate decarboxylase (EC 4.1.1.63), 3,4-dihydroxyphthalate decarboxylase (EC 4.1.1.69), phenylphosphate carboxylase (EC 4.1.1.x) (J Bacteriol. 2004 Jul;186(14):4556-67). 4-hydroxybenzoate decarboxylase, for examples the enzymes including an amino acid sequence as set forth in SEQ ID NOs: 2, 4 or 6 or an amino acid sequence encoded by the nucleotide sequence as set forth in SEQ ID NOs: 1, 3 or 5 can be preferably used. Specific examples of enzymes include Gene Ontology Number (GO No.) GO:0018799, GO:0047662, GO:0050223, GO:0050159, GO:0018798, GO:0018796, GO:0047556, GO:0047431, GO:0018862 and the like.

The enzymes are summarized together with their genes in Table 1.

**TABLE 1**

| **Enzyme** | **Gene** | **GenBank Accession No.** | **Microbial Source** |
|---|---|---|---|
| Aminobenzoate decarboxylase | hpaH | AC002919.1 | *Brucella melitensis* |
| 3-Hydroxy-2-methylpyridine-4,5-dicarboxylate 4-decarboxylase | mlr6791 | BAB53020.1 | *Rhizobium loti* (strain AFF303099) |
| 4,5-Dihydroxyphthalate decarboxylase | phtD | BAA03974.1 | *Comamonas testosterone* |
| 4-hydroxybenzoate decarboxylase | RB223 | CAD71454.1 | *Rhodopirellula baltica* |
| Protocatechuate decarboxylase | aroY | BAH20873.1 | *Klebsiella pneumoniae subsp. Pneumoniae* |
| 3,4-dihydroxyphthalate decarboxylase | phtC | AAK16538.1 | *Arthrobacter keyseri* |
| phenylphosphate carboxylase | ppcA | CAI07883.1 | *Aromatoleum aromaticum* |

As long as a biocatalyst is capable of removing the hydroxy group at para-position of the compound of Chemical Formula 3 to provide the compound of Chemical Formula 2, any biocatalyst can be used for the present invention. The examples of the biocatalyst include at least one selected from the group consisting of bile-acid 7-alpha-dehydroxylase (EC 1.17.99.5), 4-hydroxybenzoyl-CoA reductase (EC 1.3.7.9), 3-dehydroquinate hydro-lyase (EC 4.2.1.10), aldos-2-ulose dehydratase (EC 4.2.1.110), Biochim Biophys Acta. 2005, 1723(1-3):63-73), o-succinylbenzoate synthase (EC 4.2.1.113), 3-dehydroshikimate hydro-lyase (EC 4.2.1.118), prephenate hydro-lyase (EC 4.2.1.51), arogenate dehydratase (EC 4.2.1.91), scytalone 7,8-hydro-lyase (EC 4.2.1.94), and 16α-hydroxyprogesterone hydro-lyase (EC 4.2.1.98) (J Steroid Biochem Mol Biol. 1991, 38(2):257-63), but not limited thereto. The particular example of the biocatalyst may be a prephenate hydro-lyase having an amino acid sequence (PHA2) as set forth in SEQ ID NO: 8 or an amino acid sequence encoded by the nucleotide sequence as set forth in SEQ ID NO: 7.

The specific examples of the enzymes includes Gene Ontology Number(GO No.) GO:0047769, GO:0004664, GO:0046565, GO:0003855, GO:0030411, GO:0033991, GO:0043748, GO:0047455, GO:0018525, GO:0033792 and the like.

The enzymes are summarized together with their genes in Table 2.

**TABLE 2**

| **Enzyme** | **Gene** | **GenBank Accession No.** | **Microbial Source** |
|---|---|---|---|
| bile-acid 7-alpha-dehydroxylase | baiA2 | AAB61150.1 | *Eubacterium sp.* (strain VPI 12708) |
| 4-hydroxybenzoyl-CoA reductase | hcrA | CAA05038.1 | *Thauera aromatic* |
| 3-dehydroquinate hydro-lyase | aroD | ACR61804.1 | *Escherichia coli* |
| o-succinylbenzoate synthase | menC | AAA71917.1 | *Escherichia coli* |
| 3-dehydroshikimate hydro-lyase | qa-4 | CAA32750.1 | *Neurospora crassa* |
| prephenate hydro-lyase | PHA2 | DAA10245.1 | *Saccharomyces cerevisiae* |
| arogenate dehydratase | Bphy | ACC72194.1 | *Burkholderia phymatum* |
| scytalone 7,8-hydro-lyase | SDH1 | BAA34046.1 | *Magnaporthe oryzae* |

For instance, prephenate hydro-lyase (PHA2) is capable of removing the hydroxy group at para-position of the compound of Chemical Formula 3 to afford the compound of Chemical Formula 2, which can be converted to the compound of Chemical Formula 1 by 4-hydroxybenzoate decarboxylase.

The catalyst which can remove at least one substituent selected form hydroxy and C1-C4 alkoxy at position 3' and/or 5' of the benzene ring from the aromatic carboxylic acid may be an enzyme selected from the group consisting of anthranilate synthase (EC 4.1.3.27), aminodeoxychorismate lyase (EC 4.1.3.38), chorismate lyase (EC 4.1.3.40), 3-dehydroquinate hydro-lyase (EC 4.2.1.10), 3-dehydroshikimate hydro-lyase (EC 4.2.1.118), prephenate hydro-lyase (EC 4.2.1.51), 5-O-(1-carboxyvinyl)-3-phosphoshikimate phosphate-lyase (EC 4.2.3.5), isochorismate lyase (EC 4.2.99.21), and hydroxyphenylpyruvate synthase (EC 5.4.99.5), or a microorganism producing the enzyme, a lysate of the microorganism, or an extract from the microorganism cell lysate. The aminodeoxychorismate lyase (ADC lyase) may have an amino acid sequence as set forth in SEQ ID NO: 10, or an amino acid sequence encoded by the nucleotide sequence as set forth in SEQ ID NO: 9.

Examples of the catalyst having the activity to remove at least one substituent selected form hydroxy and C1-C4 alkoxy at position 3' and/or 5' of the benzene ring from the aromatic carboxylic acid include Gene Ontology Numbers (GO Nos.) GO:004049, GO:005950, GO:004107, GO:008813, GO:0046565, GO:0043904, GO:008696, GO:004664, GO:003855, GO:004106 and the like.

The enzymes are summarized, together with their genes in Table 2.

**TABLE 3**

| **Enzyme** | **Gene** | **GenBank Accession No.** | **Microbial Source** |
|---|---|---|---|
| Anthranilate synthase | TRP2 | AAA35175.1 | *Saccharomyces cerevisiae* |
| Aminodeoxychorismate lyase | ABZ2 | DAA10190.1 | *Saccharomyces cerevisiae* |
| Chorismate lyase | ubiC | CAA47181.1 | *Escherichia coli* |
| 3-Dehydroquinate hydro-lyase | aroD | ACR61804.1 | *Escherichia coli* |
| 3-Dehydroshikimate hydro-lyase | quiC | AAC37159.1 | *Acinetobacter sp.* (strain ADP1) |
| Prephenate hydro-lyase | pheA | AAA22507.1 | *Bacillus subtilis* |
| 5-O-(1-Carboxyvinyl)-3-phosphoshikimate phosphate lyase | aroC | AAA23487.1 | *Escherichia coli* |
| Isochorismate lyase | entB | AAA16102.1 | *Escherichia coli* |
| hydroxyphenylpyruvate synthase | aroH | ADE84133.1 | *Rhodobacter capsulatus* |

Generally, an enzyme may act on various substrates, and even on unknown substrates. In addition, an enzyme differs in activity from one substrate to another, and can be changed in activity or specificity for a certain substrate through modification, such as mutation or directed evolution. Like this, the enzymes of the present invention can be changed in substrate specificity or enhanced in activity using protein evolution technology so as to increase the productivity of the products.

As mentioned above, the enzymes, microorganisms as enzyme sources, lysates of the microorganisms, or extracts from the microorganism cell lysates may be used as the biocatalyst of the present invention. Contacting the substrate with the biocatalyst may be performed under a condition allowable to produce the product by bringing the substrate into contact with an enzyme, a microorganism containing the enzyme, a lysate of the microorganism, or an extract from the microorganism cell lysate, or culturing the microorganism in a medium containing the substrate.

The enzymatic reaction in each step of the present invention may be achieved by contacting the substrate with a proper enzyme or a microorganism containing the enzyme, or culturing the microorganism in a medium containing the substrate. The enzymatic reaction may be done at a pH of from 5.0 to 10.0, with an optimal pH dependent on the enzyme used. In addition, the enzymatic reaction may be done at a temperature of from 25°C to 50°C, with the optimal temperature depending on the enzyme employed. In a preferred embodiment of the present invention, conversion from aromatic aldehyde to aromatic carboxylic acid is executed at 30°C ∼ 37°C.

In a further embodiment of the present invention, the microorganism used in each step of the present invention may be recombinant or wild-type. A recombinant microorganism might be prepared by introducing a gene encoding the enzyme into a host cell using a recombinant technique.

When a recombinant enzyme is used according to one embodiment of the present invention, the process comprises 1) constructing an expression vector carrying a gene coding for the enzyme; 2) transforming the expression vector into a host cell, followed by culturing the host cell; 3) producing the enzyme from the host cell; and 4) reacting the enzyme with the substrate. The enzyme acting on the substrate may be in a pure or crude form.

Any expression vector that is employed in genetic manipulation could be applied to the construction of the recombinant expression vector for use in producing a terephthalic acid or a derivative thereof. So long as it is transformed with the recombinant expression vector to expresses the gene of interest to produce an active enzyme protein, any strain, whether bacterial, fungal, or of yeast, can be used as a host cell in the present invention. Preferred is *E. coli.*

Focusing on the synthesis of terephthalic acid from biomass, the present invention is configured to chemically or biologically degrade lignin into breakdown products from which aromatic carboxylic acid, particularly terephthalic acid or a derivative thereof is produced.

Within the scope of the lignin of the present invention are lignin, lignin derivatives, lignin fragments, and lignin-containing material. The term "lignin derivatives," as used herein, is intended to encompass lignin compounds modified by a chemical reaction, such as phenolation, acetylation, etc. The term "lignin fragments" means breakdown products obtained as a result of the chemical or biological degradation of lignin.

Typically, lignin is obtained by separating cellulose and hemicelluloses in the biorefinery or pulping process. There are various types of lignin including kraft lignin (alkaline lignin), dealkaline lignin, hydrolytic lignin, organosolv lignin, and sodium lignin sulfonate, according to production process. As a by-product from the lignocelluloses bioethanol process, lignin can be also used. Lignin is an aromatic polymer surrounding microfibers, forming a resinous structure in which phenylpropanoids, such as coumaryl alcohol, coniferyl alcohol, sinapyl alcohol, etc. serve as structural units, being polymerized via carbon-carbon bonds or carbon-oxygen bonds in a haphazard manner.

The degradation of lignin may be biodegradation or physicochemical degradation, the latter being preferred because of higher degradation rate. Biodegradation of lignin may be carried out with enzymes such as peroxidase and laccase. Additionally, lignin may be degraded physicochemically. Among the types of physicochemical degradation available for lignin in the present invention are pyrolysis, gasification, hydrogenolysis, acidolysis, alkaline lysis, chemical oxidation, and hydrolysis under supercritical condition.

In one embodiment, the acidolysis or alkaline lysis of lignin is preferably accomplished by treatment with H₂SO₄, HCl, or HNO₃ at a concentration of 0.1 to 5 % (w/v) or with a high concentration (0.5 to 2.0 mol/L) NaOH or KOH solution. Preferably, the acidic or alkaline treatment is carried out at about 80 ∼ 350°C for 5 ∼ 120 min.

Turning to pyrolysis, lignin can be degraded at as high as 350 ∼ 650°C using a high pressure reactor. The efficiency of pyrolysis can be increased in the presence of a catalyst such as nitrobenzene, KMnO₄, H₂O₂, zeolite, etc. In addition, the degradation of lignin can be accomplished using other physicochemical methods such as hydrogenolysis and hydrolysis under supercritical conditions.

The degradation of lignin is preferably carried out at an oxygen pressure of 2 - 20 bar. In addition, the degradation processes are preferably completed within 200 min, but the duration may be adjusted appropriately.

The lignin breakdown products include a mixture of aromatic monomers including aromatic aldehydes, such as vanillin, syringaldehyde, p-hydroxybenzaldehyde, and terephthalic acid such as vanillic acid, syringic acid, p-hydroxybenzoic acid, etc., and contain compounds of Chemical Formula 3 and/or Chemical Formula 4.

In accordance with an aspect thereof, the present invention addresses a process of biologically producing a terephthalic acid represented by Chemical Formula 1, or a derivative thereof, including:
degrading lignin to give a lignin breakdown product including an aromatic carboxylic acid having a p-hydroxyl group of Chemical Formula 3; and
contacting the lignin breakdown product, simultaneously or sequentially with a biocatalyst having an activity to remove a p-hydroxy group from the aromatic carboxylic acid of Chemical Formula 3, and a biocatalyst having an activity to introducing a carboxyl group at p-position of the aromatic carboxylic acid.

When X and Y in Chemical Formula 3 are not simultaneously hydrogen, the method may further include applying to the aromatic carboxylic acid a biocatalyst having an activity to remove at least one substituent selected from hydroxy and C1-C4 alkoxy at either or both positions 3 and 5, before, simultaneously with or after the contact with the biocatalyst having an activity to remove a p-hydroxy group and/or the biocatalyst having an activity to introducing p-carboxyl group.

In addition, when the lignin breakdown product contains an aromatic aldehyde compound of Chemical Formula 4, such as vanillin, syringaldehyde, etc., the process may further include converting the lignin breakdown product into the compound of Chemical Formula 3 by oxidation, prior to or after the contact of the lignin breakdown product including the compound of Chemical Formula 3 with the biocatalyst having an activity to remove p-hydroxy from the compound of Chemical Formula 3.

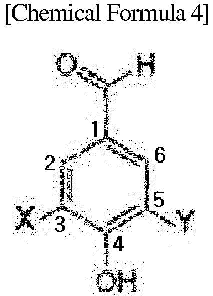

For example, conversion from the aromatic aldehyde of Chemical Formula 4 to the aromatic carboxylic acid of Chemical Formula 3 may be performed by chemical or biological reaction.

For chemical conversion, a silver oxide method or a caustic fusion method may be utilized. First, aromatic monomers with an aldehyde functional group are reacted with 1 M NaOH at 55 ∼ 60°C for about 10 min in the presence of 1 M Ag₂O, followed by neutralization with the equal amount of 1 M HCl with agitation to afford the terephthalic acid as a precipitate.

The biological reaction is performed by using a biocatalyst such as an enzyme, a whole microbial cell, a microbial cell lysate, or a cell extract. The enzyme useful in the present invention may be exemplified by aldehyde dehydrogenase (EC 1.2.1.3, EC 1.2.1.4, EC 1.2.1.5), vanillin dehydrogenase (EC 1.2.1.67) and other enzymes functionally corresponding thereto. Non-limiting examples of these enzymes include GenBank ID CAD60262.1, ABK09332.1, Uniprot ID P47771, and P54114.

The enzymatic reactions in the presence of a pure enzyme as well as a microbial whole cells expressing the enzyme or functionally identical enzymes, such as *Saccharomyces cerevisiae, Bacillus subtilis, Escherichia coli, Pseudomonas fluorescens, Pseudomonas putida, Serratia marcescens, Sphingomonas paucimobilis, Streptomyces viridosporus, Desulfovibrio vulgaris,* or *Burkholderia cepacia,* or a lysate or extract thereof are considered as the enzymatic reaction. The aldehyde dehydrogenase may have an amino acid sequence (ALD4) as set forth in SEQ ID NO: 12 or an amino acid sequence encoded by the nucleotide sequence as set forth in SEQ ID NO: 11.

The terephthalic acid can be converted into derivative thereof which is in turn polycondensed with diols to manufacture polyester such as polyethylene terephthalate (hereinafter referred to as "PET") or polytrimethylene terephthalate (hereinafter referred to as "PTT"). Superior in strength, hygroscopicity, and wrinkle recovery, PET is widely used in synthetic fibers. In addition, PET is used in a variety of packaging products due to its transparency, mechanical properties, and a gas barrier properties. PET is used in applications involving packing foods, soft drinks, alcoholic beverages, detergents, cosmetics, drugs, and edible oils.

By using a biological process in which a substrate including an aromatic carboxylic acid, or a derivative thereof is contacted with a biocatalyst having an activity to introducing p-carboxyl group, the present invention allows terephthalic acid or a derivative thereof to be produced in an environment-friendly manner and at higher specificity, compared to a chemical process. The terephthalic acid or a derivative thereof can be obtained from lignin by degrading the lignin to give lignin breakdown products including aromatic monomers, followed by biological conversion of the breakdown products into terephthalic acid or a derivative thereof.

A better understanding of the present invention may be obtained through the following examples which are set forth to illustrate the present invention.

### <Analysis of Aromatic Monomers>

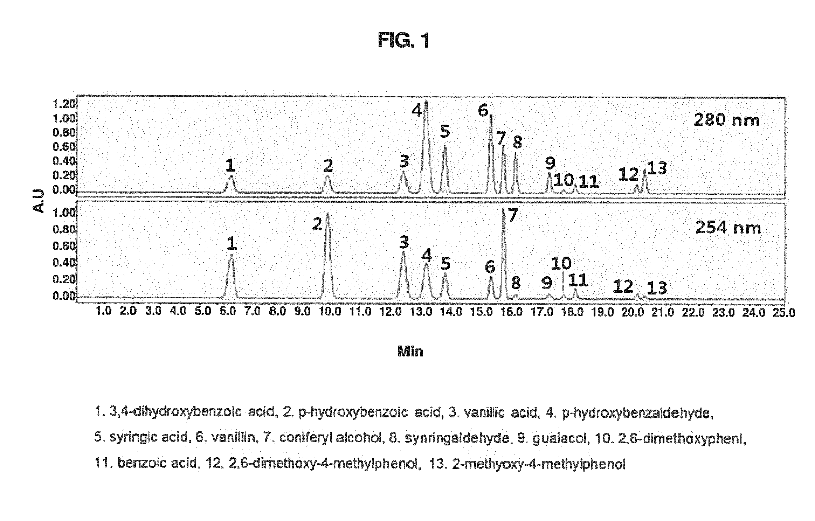

For use in the quantitative analysis of lignin-derived aromatic monomers, standard solutions of enzymatic reaction intermediates including p-hydroxybenzoic acid, benzoic acid and terephthalic acid were prepared, and analyzed using Waters e2695 HPLC equipped with a Waters 2489 UV/VIS (254 nm, 280 nm) detector (FIG. 1). For analysis, an XBridge C18 column (4.6 x 150 mm, 5 µm) was employed, and maintained at 35°C, with a mobile phase moved at 1 mL/min. The mobile phase was a mixture of A) a 5% acetonitrile solution containing 0.1% formic acid, and B) a 50% acetonitrile solution containing 0.1 % formic acid, and was applied in the following gradient elution manner: 1.5 min (0% B), 9.5 min (90% B), 16.5 min (40% B), 21.5 min (24% B), and 24.5 min (0% B). Prior to subsequent analysis, the column was pre-equilibrated for 6 min.

The production of terephthalic acid was monitored using HPLC and ESI-MS/MS (Waters TQD). This HPLC was conducted in the same condition as in the above HPLC. The condition for mass spectrometry was optimized with a standard solution of terephthalic acid. Mass spectra were obtained in the positive mode and the optimal condition for the spectrometry was set forth as follows: Capillary voltage: 3 kV, Cone voltage: 25 V, Source temperature: 120°C, Desolvation temperature: 300°C, Desolvation gas flow: 600 L/hr (N2), and Cone gas flow: 60 L/hr (N2). On the HPLC-ESI-MS/MS spectra, a peak for terephthalic acid was detected, in comparison with the standard solution, in the scan mode (50 ∼ 200, m/z) as molecular ions and specific fragment ions were generated at a given collision energy.

### [EXAMPLE 1] Production of Benzoic Acid by Dehydroxylation

### <Step 1> Construction of Recombinant Expression Vector Carrying Prephenate Hydro-lyase (PHA2) Gene and Preparation of Transformed Microorganism

To produce prephenate hydro-lyase (PHA2), a PHA2 gene from *S*. *cerevisiae* was cloned. First, genomic DNA was isolated from *S*. *cerevisiae* ATCC 204508. On the basis of a nucleotide sequence (GenBank Accession Number; CAA86380.1) coding for a PHA2 gene, the following primers were designed:
Forward primer 1 (SEQ ID NO: 13):
   5'-AAACATATG AAAATAAAAATTTTAGTAGA -3'
Reverse primer 1 (SEQ ID NO: 14):
   5'-AAACTCGAG TTTGTGATAATATCTCTCAT -3'

The nucleotide sequence of PHA2 gene was amplified by PCR using the primers, with the genomic DNA of *S*. *cerevisiae* ATCC 204508 serving as a template.

A total volume of 50 µl of a PCR composition contained 100 ng of the template, 10 pmol of each primer, 2.5 mM dNTPs, a 1x PCR buffer, and a 2.5 U Taq polymerase. PCR started with pre-denaturation at 94°C for 5 min, and was performed with 30 cycles of denaturation at 95°C for 1 min; annealing at 54°C for 30 sec; and elongation at 72°C for 2 min, followed by post-polymerization at 72°C for 5 min for final elongation.

The PCR product thus obtained was digested with NdeI/XhoI restriction enzymes, and inserted in the presence of T4 DNA ligase into the plasmid vector pET28a (Novagen) which was previously cut with the same enzymes, to construct a recombinant pET28a/PHA2 vector. PCR and cloning results were monitored by 1.2 % agarose electrophoresis.

The recombinant expression vector was typically transformed into E. coli BL21 (DE3), and the transformant was cryo-preserved in 15 % glycerol until use for enzyme expression.

### <Step 2> Production of Prephenate Hydro-lyase

To produce prephenate hydro-lyase in a large amount, the cryo-preserved recombinant E. coli was inoculated into 5 mL of LB broth in a test tube, and seed cultured at 37°C with agitation to an absorbance of 2.5 at 600 nm. Then, the seed culture was added to 100 mL of LB broth in a 300 mL flask and cultured. When absorbance at 600 nm reached 0.6, 1 mM IPTG was added to induce the expression of the enzyme. In this regard, the cells were cultured at 30°C with agitation at 250 rpm, and further incubated for 10 hrs after IPTG addition.

Then, the transformed cell culture was centrifuged at 4,000x g and 4°C for 30 min, washed twice with a PBS buffer, mixed with a 50 mM Tris-HCl buffer (pH 7.5) before ultrasonic disruption. The cell lysate was again centrifuged at 13,000×g and 4°C for 20 min, and the supernatant was withdrawn and subjected to Ni-NTA His-Tag chromatography to separate the enzyme. The bound enzyme was eluted with a 50 mM Tris-HCl buffer (pH 7.5) using a centrifugal separation filter (10 kDa). After concentration, the eluate was quantitatively analyzed using a protein assay (Bradford). Finally, the enzyme was obtained at a concentration of 5 mg/mL, and used in a enzymatic reaction with p-hydroxybenzoic acid as a substrate. Proteins were analyzed on 12 % polyacrylamide gel by electrophoresis.

### <Step 3> Production of Benzoic acid

To 0.5 mL of a mixture containing 10 mM p-hydroxybenzoic acid (Sigma), 1 mM EDTA, 20 mM 2-mercaptoethanol, 50 µL of the 5 mg/L prephenate hydro-lyase purified in step 2 was added. After reaction for 5 hrs in a 37°C incubator, three volumes of 1 N sodium hydroxide were added to the reaction mixture which was then filtered through a syringe filter (0.22 µm) to remove impurities. Concentrations of p-hydroxybenzoic acid and benzoic acid in the resulting sample were monitored using HPLC, and the results are given in Table 4.

**TABLE 4**

| | Before enzyme reaction (mM) | After enzyme reaction (mM) |
|---|---|---|
| p-hydroxybenzoic acid | 9.98 | 2.46 |
| benzoic acid | 0.00 | 6.12 |

As shown in Table 4, p-hydroxybenzoic acid was converted into benzoic acid by prephenate hydro-lyase. The production of benzoic acid was confirmed by the mass spectroscopic analysis.

### [EXAMPLE 2] Production of Terephthalic acid by the carboxylation reaction

### <Step 1> Construction of Recombinant Expression Vector Carrying 4-Hydroxybenzoic acid decarboxylase-coding Gene and Preparation of Transformed Microorganism

To produce 4-hydroxybenzoic acid decarboxylase, genes of bsdB, bsdC and bsdD derived from *B.substilis (strain 168)* were cloned. It was reported that 4-Hydroxybenzoic acid decarboxylase catalyzed reversible reaction of the forward and reverse reaction depending on the reacting condition and the substrate (Can. J. Microbiol. 54: 75-81(2008)). 4-Hydroxybenzoic acid decarboxylase was used for introducing the carboxyl group at 4-position of benzoic acid in this test. First, genomic DNA was isolated from *B.substilis* ATCC 6051. On the basis of a nucleotide sequence (GenBank Accession Number; bsdB:BAA08996.1., bsdC: BAA08997.1., bsdD: CAX52546.1) coding for the enzyme, the following primers were designed. 4-Hydroxybenzoic acid decarboxylase is a complex consisting of three kinds of domains of bsdB, bsdC and bsdD which are polycistronic gene located sequentially on the chromosome. Thus, the genes were cloned by using the following primers:
Forward primer 2 (SEQ ID NO: 15):
   5'-AAACATATG AAAGCAGAATTCAAGCGTAA -3'
Reverse primer 2 (SEQ ID NO: 16):
   5'-AAACTCGAG AGCCTTTCGTTCCGGCACCG -3'

The nucleotide sequence of the gene was amplified by PCR using the primers, with the genomic DNA of *B. subtilis* ATCC 6051 serving as a template.

A total volume of 50 µl of a PCR composition contained 100 ng of the template, 10 pmol of each primer, 2.5 mM dNTPs, a 1x PCR buffer, and a 2.5 U Taq polymerase. PCR started with pre-denaturation at 94°C for 5 min, and was performed with 30 cycles of denaturation at 94°C for 1 min; annealing at 55°C for 30 sec; and elongation at 72°C for 3 min, followed by post-polymerization at 72°C for 5 min for final elongation.

The PCR product thus obtained was digested with NdeI/XhoI restriction enzymes, and inserted in the presence of T4 DNA ligase into the plasmid vector pET28a (Novagen) which was previously cut with the same enzymes, to construct a recombinant pET28a/PAD vector. PCR and cloning results were monitored by 1.5 % agarose electrophoresis.

The recombinant expression vector was typically transformed into *E. coli* BL21 (DE3), and the transformant was cryo-preserved in 15 % glycerol until use for enzyme expression.

### <Step 2> Production of the Carboxylase

To produce the carboxylase in a large amount, the cryo-preserved recombinant *E. coli* was inoculated into 5 mL of LB broth in a test tube, and seed cultured at 37°C with agitation to an absorbance of 2.5 at 600 nm. Then, the seed culture was added to 100 mL of LB broth in a 300 mL flask and cultured. When absorbance at 600 nm reached 0.6, 0.5 mM IPTG was added to induce the expression of the enzyme. In this regard, the cells were cultured at 33°C with agitation at 250 rpm, and further incubated for 6 hrs after IPTG addition.

Then, the transformed cell culture was centrifuged at 4,000x g and 4°C for 20 min, washed twice with a PBS buffer, mixed with a 50 mM Tris-HCl buffer (pH 7.5) before ultrasonic disruption. The cell lysate was again centrifuged at 13,000×g and 4°C for 20 min, and the supernatant was withdrawn and subjected to Ni-NTA His-Tag chromatography to purify the enzyme. The bound enzyme was eluted with a 50 mM Tris-HCl buffer (pH 7.5) using a centrifugal separation filter (10 kDa). After concentration, the eluate was quantitatively analyzed using a protein assay (Bradford). Finally, the enzyme was obtained at a concentration of 5 mg/mL, and used in enzymatic reaction.

### <Step 3> Production of Terephthalic acid

To 0.5 mL of a mixture containing 10 mM benzoic acid (Sigma), 100 mM sodium bicarbonate, 50µL of the 5 mg/L the Carboxylase purified in step 2 was added and then reacted for 5 hours in the 37°C anaerobic chamber. After reaction termination, three volumes of aqueous solution of 1 N sodium hydroxide were added to the reaction mixture which was then filtered through a syringe filter (0.22 µm) to remove impurities. Concentrations of reduced benzoic acid and produced terephthalic acid in the resulting sample were monitored using HPLC, and the results are given in Table 5.

**TABLE 5**

| | Before enzyme reaction (mM) | After enzyme reaction (mM) |
|---|---|---|
| benzoic acid | 10.32 | 4.81 |
| Terephthalic acid | 0.00 | 3.24 |

As shown in Table 5, benzoic acid was converted into terephthalic acid by 4-Hydroxybenzoic acid decarboxylase. The production of terephthalic acid was confirmed by the mass spectroscopic analysis.

### [EXAMPLE 3] Production of Terephthalic acid from p-hydroxybenzoic acid

The one-pot enzyme reaction using the Dehydroxylase in Example 1 and the Carboxylase in Example 2 are used for terephthalic acid from p-hydroxybenzoic acid.

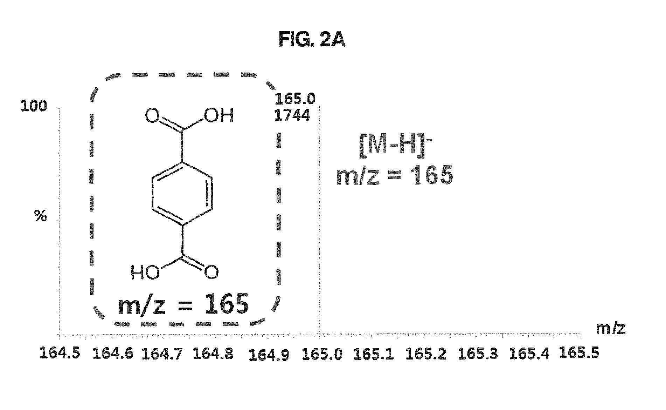

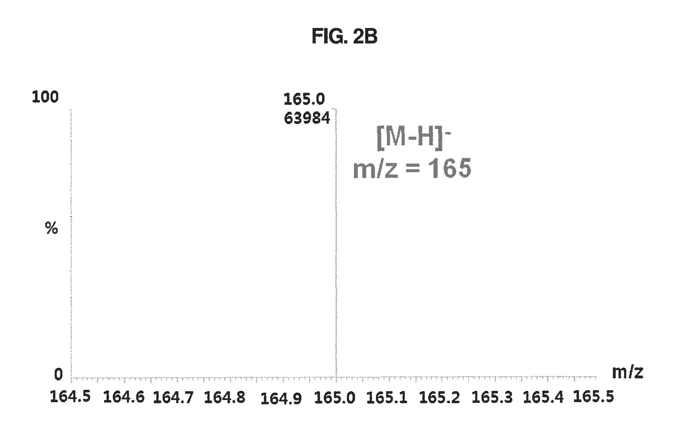

50 µL(5mg/mL) each of the dehydroxylase in Example 1 and the Carboxylase in Example 2 was to 0.5 mL of a mixture containing 400 µL of 20 mM p-hydroxybenzoic acid (Sigma) dissolved in 100 mM of sodium bicarbonase (pH 8.5), and then reacted for 5 hrs in a 37°C incubator. After the reaction termination, three volumes of 1 N sodium hydroxide were added to the reaction mixture which was then filtered through a syringe filter (0.22 µm) to remove impurities. Concentrations of reduced p-hydroxybenzoic acid and produced terephthalic acid in the resulting sample were monitored using HPLC, and the results are given in Table 6. The produced terephthalic acid was confirmed by mass spectrometry (FIGS. 2A-2B).

**TABLE 6**

| | Before enzyme reaction (mM) | After enzyme reaction (mM) |
|---|---|---|
| p-hydroxybenzoic acid | 19.59 | 2.93 |
| benzoic acid | 0.00 | 3.48 |
| Terephthalic acid | 0.00 | 7.29 |

### [EXAMPLE 4] Production of aminodeoxychorismate lyase (ADC lyase)

### <Step 1> Construction of Recombinant Expression Vector Carrying Aminodeoxychorismate Lyase Gene and Preparation of Transformed Microorganism

To produce aminodeoxychorismate lyase (ADC lyase), an ADC lyase gene from *S. cerevisiae* was cloned. First, genomic DNA was isolated from *S*. *cerevisiae* KCCM 50712. On the basis of a nucleotide sequence (GenBank Accession Number; DAA10190.1) coding for the ADC lyase of S. *cerevisiae* KCCM 50712, the following primers were designed:
Forward primer 3 (SEQ ID NO: 17):
   5'-AAACATATG TCACTAATGGACAATTGGAA-3
Reverse primer 3 (SEQ ID NO: 18):
   5'- AAACTCGAG ATATTTTGTCTTCACTGTTC-3'

The nucleotide sequence of ADC lyase gene was amplified by PCR using the primers, with the genomic DNA of *S*. *cerevisiae* KCCM 50712 serving as a template.

A total volume of 50 µl of a PCR composition contained 100 ng of the template, 10 pmol of each primer, 2.5 mM dNTPs, a 1x PCR buffer, and a 2.5 U Taq polymerase. PCR started with pre-denaturation at 94°C for 5 min, and was performed with 30 cycles of denaturation at 94°C for 1 min; annealing at 55°C for 30 sec; and elongation at 72°C for 3 min, followed by post-polymerization at 72°C for 5 min for final elongation.

The PCR product thus obtained was digested with NdeI/XhoI restriction enzymes, and inserted in the presence of T4 DNA ligase into the plasmid vector pET28a (Novagen) which was previously cut with the same enzymes, to construct a recombinant pET28a/ADCL vector. PCR and cloning results were monitored by 1.2 % agarose electrophoresis.

The recombinant expression vector was typically transformed into *E. coli* BL21 (DE3), and the transformant was cryo-preserved in 15 % glycerol until use for enzyme expression.

### <Step 2> Production of ADC Lyase

To produce ADC lyase in a large amount, the cryo-preserved recombinant *E. coli* was inoculated into 5 mL of LB broth in a test tube, and seed cultured at 37°C with agitation to an absorbance of 2.5 at 600 nm. Then, the seed culture was added to 100 mL of LB broth in a 300 mL flask and cultured. When absorbance at 600 nm reached 0.6, 0.5 mM IPTG was added to induce the expression of the enzyme. In this regard, the cells were cultured at 33°C with agitation at 250 rpm, and further incubated for 6 hrs after IPTG addition.

Then, the transformed cell culture was centrifuged at 4,000x g and 4°C for 20 min, washed twice with a PBS buffer, mixed with a 50 mM Tris-HCl buffer (pH 7.5) before ultrasonic disruption. The cell lysate was again centrifuged at 13,000×g and 4°C for 20 min, and the supernatant was withdrawn and subjected to Ni-NTA His-Tag chromatography to purify the enzyme. The bound enzyme was eluted with a 50 mM Tris-HCl buffer (pH 7.5) using a centrifugal separation filter (10 kDa). After concentration, the eluate was quantitatively analyzed using a protein assay (Bradford). Finally, the enzyme was obtained at a concentration of 5 mg/mL, and used in enzymatic reaction.

### [EXAMPLE 5] Production of Terephthalic acid by Enzymatic Reaction Using Lignin Degradation Product as Substrate

Lignin was degraded using a laboratory high-pressure reactor (450 mL, Parr 4562). A reactant with a lignin content of 5.0 % (w/v) was prepared by adding 10.0 g of kraft lignin to 200 mL of 1 M NaOH. The reactant was further mixed with 10 g of the catalyst KMnO₄, loaded to a stainless steel high-pressure reactor with an internal volume of 450 mL, sealed, and stirred at a speed of 500 rpm. After the reactor was filled with oxygen gas at a pressure of about 5 bar for 2 min via a sampling line communicating with the interior thereof, it was heated. When the internal temperature of the reactor reached 140°C, the reaction was continued for 60 min. The reaction temperature was adjusted by a PID controller through a cooling water tube. At 60 min of the reaction, a sample was withdrawn via a sampling line, and then, the reaction was terminated.

2 mL of the sample containing alkaline breakdown products of lignin was 3-fold diluted in 4 mL of distilled water, followed by removal of lignin by filtration (10 kDa MWCO). To 1 mL of the lignin-free sample were added 9 volumes of methanol, and lignin breakdown products were purified by filtration through a syringe filter (0.22 µm).

The lignin breakdown products obtained above were enzymatically converted into terephthalic acid. In this regard, a solution of the lignin breakdown products was adjusted to pH 8.0 using a small amount of 10 M HCl, and filtered through a 10 kDa MWCO filter. The filtrate was used as a substrate in reaction with a mixture of 50 µL of 5 mg/mL aminodeoxychorismate lyase (ADC lyase), obtained in Example 4, and 50 µL of 5mg/ml aldehyde dehydrogenase (Sigma A6338, ALD4) for 3 hrs in a 37°C incubator. Of the reaction mixture, 50 µL was withdrawn and used for analysis while the remainder was used as a substrate for a subsequent enzyme reaction. For p-hydroxybenzoic acid analysis, the sample was extracted with 9 volumes of methanol (450 µL), and filtered through a syringe filter (0.22 µm). The resulting sample was analyzed for p-hydroxybenzoic acid by HPLC (Table 7).

The Dehydroxylase of Example 1 and the Carboxylase of Example 2 was added at a final concentration of 500 µg/mL respectively, to 400 µL of the remainder enzyme reaction mixture which was then mixed with sodium bicarbonate at a final concentration of 100 mM, followed by reaction for 10 hrs in a 37°C anaerobic chamber. After completion of the reaction, the sample was filtered through a syringe filter (0.22 µm) to remove impurities. Analysis results of the sample are summarized in Table 7.

**TABLE 7**

| Compound | Lignin degradation Product (mM) | Product obtained after treatment with aldehyde dehydroganase and ADC lyase (mM) | Product obtained after treatment with dehydroxylase and Carboxylase (mM) |
|---|---|---|---|
| p-Hydroxybenzoic acid | 0.21 | 4.53 | 1.35 |
| p-Hydroxybenzaldehyde | 0.91 | 0.18 | 0.00 |
| Vanillic acid | 2.40 | 1.95 | 1.24 |
| Vanillin | 5.82 | 2.98 | 1.32 |
| Syringic acid | 0.50 | 0.12 | 0.00 |
| Syrinaldehyde | 0.21 | 0.00 | 0.00 |
| Benzoic acid | 0.00 | 0.00 | 0.52 |
| Terephthalic acid | 0.00 | 0.00 | 1.16 |

## Claims

1. A method of biologically producing an terephthalic acid or a derivative thereof represented by Chemical Formula 1, comprising contacting a substrate containing an aromatic carboxylic acid represented by Chemical Formula 2 with a biocatalyst that adds a carboxyl group at para-position of the aromatic carboxylic acid: 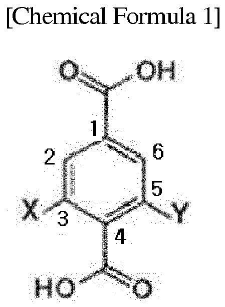 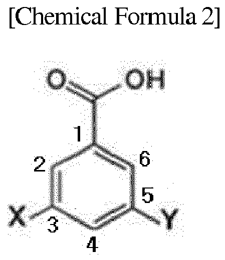 wherein, X and Y are independently hydrogen, hydroxy, or C₁-C₄ alkoxy.

2. The method of claim 1, wherein the substrate also comprises an aromatic carboxylic acid of Chemical Formula 3 having a para-hydroxy group, and the method further comprises contacting the substrate with a biocatalyst that removes the para-hydroxy group from the aromatic carboxylic acid of Chemical Formula 3, before, simultaneously with, or after contacting with the biocatalyst that adds a carboxyl group at para-position of the aromatic carboxylic acid of Chemical Formula 2: 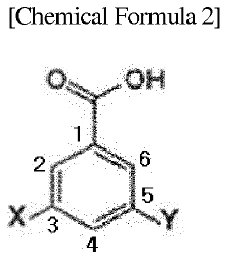 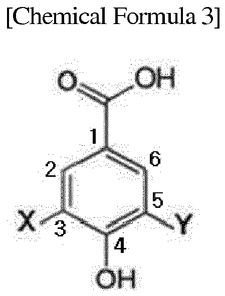 wherein, X and Y are independently hydrogen, hydroxy, or C₁-C₄ alkoxy.

3. The method of claim 1, wherein the substrate comprises an aromatic aldehyde of Chemical Formula 4, and the method further comprises
converting the aromatic aldehyde of Chemical Formula 4 to an aromatic carboxylic acid of Chemical Formula 3 by chemical oxidation or biocatalytic oxidation before or simultaneously with contacting with the biocatalyst that removes the para-hydroxy group;
and contacting the aromatic carboxylic acid of Chemical Formula 3 with a biocatalyst that removes the para-hydroxy group from the aromatic carboxylic acid of Chemical Formula 3 to provide the aromatic carboxylic acid of Chemical Formula 2: 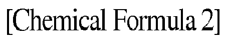 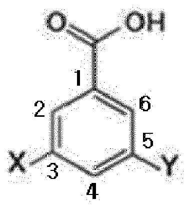 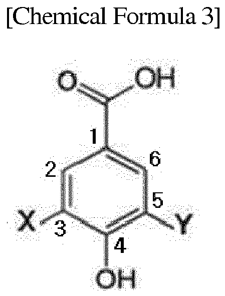 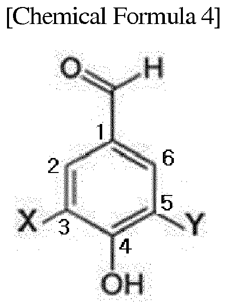 wherein, X and Y are independently hydrogen, hydroxy, or C₁-C₄ alkoxy.

4. The method of claim 1 comprising
contacting a substrate containing an aromatic carboxylic acid of Chemical Formula 3 having a para-hydroxy group with a biocatalyst that removes the para-hydroxy group to provide an aromatic carboxylic acid of Chemical Formula 2 for producing the substrate containing an aromatic carboxylic acid represented by Chemical Formula 2.

5. The method of claim 4 comprising
providing a substrate comprising an aromatic aldehyde of Chemical Formula 4;
converting the aromatic aldehyde of Chemical Formula 4 to an aromatic carboxylic acid of Chemical Formula 3 by chemical oxidation or biocatalytic oxidation for providing the substrate containing an aromatic carboxylic acid of Chemical Formula 3.

6. The method of claim 3 or 5, wherein
(i) the conversion is performed utilizing a silver oxide method or a caustic fusion method, or
(ii) the biocatalytic oxidation is performed using an enzyme, a whole microbial cell, a microbial cell lysate, or a cell extract, preferably using aldehyde dehydrogenase or vanillin dehydrogenase.

7. The method of any one of claims 2 to 6, the compounds represented by Chemical Formula 3 and Chemical Formula 4 are derived from lignin, wherein the substrate containing an aromatic carboxylic acid represented by Chemical Formula 2, is preferably obtained by:
degrading lignin to give a lignin degradation product comprising an aromatic carboxylic acid having a para-hydroxy group of Chemical Formula 3; and
contacting the lignin degradation product with the biocatalyst that removes a para-hydroxy group from the benzene ring of Chemical Formula 3 to provide an aromatic carboxylic acid represented by Chemical Formula 2.

8. The method of claim 7, wherein the degradation of lignin is carried out using at least one selected from the group consisting of pyrolysis, gasification, hydrogenolysis, acidolysis, alkaline lysis, chemical oxidation, hydrolysis under supercritical conditions, and enzymolysis.

9. The method of claim 7 or 8, wherein the lignin breakdown products include a mixture of aromatic monomers including aromatic aldehydes, aromatic carboxylic acids, preferably compounds of Chemical Formula 2 and/or 3 and/or Chemical Formula 4.

10. The method of any one of claims 1 to 9, wherein X and Y are not simultaneously hydrogen in Chemical Formula 1, 2, 3 or 4, and the method further comprises contacting the substrate with a biocatalyst that removes a substituent selected from the group consisting of hydroxy and C₁-C₄ alkoxy located at positions 3, position 5, or both of positions 3 and 5 of the benzene ring, before, simultaneously with, or after contacting with the biocatalyst that removes the para-hydroxy group, or before, simultaneously with, or after contacting with the biocatalyst that adds a carboxyl group at para-position of the aromatic carboxylic acid, or before, simultaneously with, or after the oxidation of the aromatic aldehyde of Chemical Formula 4.

11. The method of any one of claims 1 to 10, wherein the biocatalyst is an enzyme, a microorganism that produces the enzyme, a lysate of the microorganism, or an extract from the lysate of the microorganism, wherein the biocatalyst is preferably a wild-type microorganism or a recombinant microorganism.

12. The method of any one of claims 1 to 11, wherein contacting the substrate with the biocatalyst comprises contacting the substrate with an enzyme, a microorganism containing the enzyme, a lysate of the microorganism, or an extract from the lysate of the microorganism, or by culturing the microorganism in a medium containing the substrate.

13. The method of any one of claims 1 to 12, wherein
- the biocatalyst that adds a carboxyl group at the para-position of the aromatic carboxylic acid comprises at least one enzyme selected from the group consisting of aminobenzoate decarboxylase (EC 4.1.1.24), 3-Hydroxy-2-methylpyridine-4,5-dicarboxylate 4-decarboxylase (EC 4.1.1.51), 4,5-Dihydroxyphthalate decarboxylase (EC 4.1.1.55), Orsellinate decarboxylase (EC 4.1.1.58, Gallate decarboxylase (EC 4.1.1.59), 4-hydroxybenzoate decarboxylase (EC 4.1.1.61), Protocatechuate decarboxylase (EC 4.1.1.63), 3,4-dihydroxyphthalate decarboxylase (EC 4.1.1.69) phenylphosphate carboxylase (EC 4.1.1.x); a microorganism that produces at least one of the foregoing enzymes; a lysate of a microorganism containing at least one of the foregoing enzymes; or an extract from the lysate of the microorganism containing at least one of the foregoing enzymes; and/or
- the biocatalyst that removes para-hydroxy comprises at least one enzyme selected from the group consisting of bile-acid 7-alpha-dehydroxylase (EC 1.17.99.5), 4-hydroxybenzoyl-CoA reductase (EC 1.3.7.9), 3-dehydroquinate hydro-lyase (EC 4.2.1.10), aldos-2-ulose dehydratase (EC 4.2.1.110), o-succinylbenzoate synthase (EC 4.2.1.113), 3-dehydroshikimate hydro-lyase (EC 4.2.1.118), prephenate hydro-lyase (EC 4.2.1.51), arogenate dehydratase (EC 4.2.1.91), scytalone 7,8-hydro-lyase (EC 4.2.1.94), 16α-hydroxyprogesterone hydro-lyase (EC 4.2.1.98); a microorganism that produces at least one of the foregoing enzymes; a lysate of a microorganism containing at least one of the foregoing enzymes; or an extract from the lysate of the microorganism containing at least one of the foregoing enzymes; and/or
- the biocatalyst that removes at least one substituent selected from the group consisting of hydroxy and C1-C4 alkoxy is at least one enzyme selected from the group consisting of anthranilate synthase (EC 4.1.3.27), aminodeoxychorismate lyase (EC 4.1.3.38), chorismate lyase (EC 4.1.3.40), 3-dehydroquinate hydro-lyase (EC 4.2.1.10), 3-dehydroshikimate hydro-lyase (EC 4.2.1.118), prephenate hydro-lyase (EC 4.2.1.51), 5-O-(1-carboxyvinyl)-3-phosphoshikimate phosphate-lyase (EC 4.2.3.5), isochorismate lyase (EC 4.2.99.21), hydroxyphenylpyruvate synthase (EC 5.4.99.5); a microorganism that produces at least one of the foregoing enzymes; a lysate of a microorganism containing at least one of the foregoing enzymes; or an extract from the lysate of the microorganism containing at least one of the foregoing enzymes.

14. The method of any one of claims 1 to 13, wherein
- the aromatic carboxylic acid represented by Chemical Formula 1 is at least one of a terephthalic acid, m-hydroxy terephthalic acid, 3,5-dihydroxy terephthalic acid, 3-methoxy terephthalic acid, 3,5-dimethoxy terephthalic acid, and the like; and/or
- the compound of Chemical Formula 2 is at least one of benzoic acid, m-hydroxybenzoic acid, 3,5-dihydroxybenzoic acid, 3-methoxybenzoic acid, 3,5-dimethoxybenzoic acid, and the like; and/or
- the compound of Chemical Formula 3 is at least one of 4-hydroxybenzoic acid, 3,4-dihydroxybenzoic acid, 4,5-dihydroxybenzoic acid, 3,4,5-trihydroxybenzoic acid, 3-methoxy-4-hydroxybenzoic acid, 4-hydroxy-5-methoxybenzoic acid, and 3,5-dimethoxy-4-hydroxybenzoic acid; and/or
- the compound of Chemical Formula 4 is at least one of p-hydroxybenzaldehyde, vanillin, and syringaldehyde.

15. A method of producing a polyester, wherein the polyester is produced by producing a terephthalic acid or derivatives thereof according to the method of any one of claims 1 to 14 and polycondensing the terephthalic acid or derivatives thereof with a diol.


Sequence Listing

<110>    SAMSUNG ELECTRONICS CO., LTD.

<120>    PROCESS OF BIOLOGICALLY PRODUCING A TEREPHTHALIC ACID AND
         DERIVATIVES THEREOF

<130>    EP91518FZTRpau

<140>    not yet assigned
<141>    2012-11-30

<150>    10-2012-0138374
<151>    2012-11-30

<160>    18

<170>    KopatentIn 2.0

<210>    1
<211>    615
<212>    DNA
<213>    Bacillus subtilis

<400>    1
atgaaagcag aattcaagcg taaaggaggg ggcaaagtga aactcgttgt cggaatgaca    60

ggggcaacag gggccatttt cggggtcagg ctgctgcagt ggctgaaggc cgccggagtg   120

gaaacccatc tcgttgtgtc tccttgggca aacgtcacga tcaaacacga aacaggctat   180

acgttacaag aagtagaaca actggccaca tacacttact cacataagga tcaggcggca   240

gccatttcaa gcgggtcgtt tgataccgat ggaatgattg ttgcgccgtg cagcatgaaa   300

tctctcgcaa gcattcgcac aggaatggcg gataatctgc tgacacgtgc ggcggatgtc   360

atgctcaagg agagaaaaaa actcgtcctc ttaacgagag agacgccttt gaaccaaatt   420

catctcgaaa atatgctagc gcttacgaaa atgggcacca tcattcttcc tccgatgccg   480

gcattttata atcggccgag aagcttagag gaaatggttg accatattgt ttttagaacg   540

ttggaccaat tcggcattcg gcttcctgaa gcgaagcgct ggaatgggat tgaaaaacaa   600

aaaggaggag cttga                                                    615


<210>    2
<211>    204
<212>    PRT
<213>    Bacillus subtilis

<400>    2
Met Lys Ala Glu Phe Lys Arg Lys Gly Gly Gly Lys Val Lys Leu Val
  1               5                  10                  15

Val Gly Met Thr Gly Ala Thr Gly Ala Ile Phe Gly Val Arg Leu Leu
             20                  25                  30

Gln Trp Leu Lys Ala Ala Gly Val Glu Thr His Leu Val Val Ser Pro
         35                  40                  45

Trp Ala Asn Val Thr Ile Lys His Glu Thr Gly Tyr Thr Leu Gln Glu
50                  55                  60

Val Glu Gln Leu Ala Thr Tyr Thr Tyr Ser His Lys Asp Gln Ala Ala
65                  70                  75                  80

Ala Ile Ser Ser Gly Ser Phe Asp Thr Asp Gly Met Ile Val Ala Pro
                85                  90                  95

Cys Ser Met Lys Ser Leu Ala Ser Ile Arg Thr Gly Met Ala Asp Asn
            100                 105                 110

Leu Leu Thr Arg Ala Ala Asp Val Met Leu Lys Glu Arg Lys Lys Leu
        115                 120                 125

Val Leu Leu Thr Arg Glu Thr Pro Leu Asn Gln Ile His Leu Glu Asn
    130                 135                 140

Met Leu Ala Leu Thr Lys Met Gly Thr Ile Ile Leu Pro Pro Met Pro
145                 150                 155                 160

Ala Phe Tyr Asn Arg Pro Arg Ser Leu Glu Glu Met Val Asp His Ile
                165                 170                 175

Val Phe Arg Thr Leu Asp Gln Phe Gly Ile Arg Leu Pro Glu Ala Lys
            180                 185                 190

Arg Trp Asn Gly Ile Glu Lys Gln Lys Gly Gly Ala
        195                 200

<210>    3
<211>    1422
<212>    DNA
<213>    Bacillus subtilis

<400>    3
atggcttatc aagatttcag agaatttctc gctgcccttg aaaaagaagg acagctgctt    60

acagtgaatg aagaggtaaa gccggaaccg gatttagggg cctccgcacg ggcagccagc   120

aatcttggcg ataaaagccc tgcgctctta tttaacaaca tttacggcta tcataacgcg   180

cgaattgcga tgaatgtcat cggctcttgg ccaaaccatg ccatgatgct gggcatgccg   240

aaagacacac cggtaaaaga acagtttttt gaattcgcaa agcgttatga ccagtttccg   300

atgccggtca aacgtgagga aacagcgcca tttcatgaaa atgaaatcac agaagatatc   360

aatttgttcg atatactgcc tcttttcaga attaaccagg gtgatggagg ctactattta   420

gacaaagcat gtgtcatttc ccgtgatctt gaggaccctg acaacttcgg caaacaaaat   480

gtcggcattt acagaatgca agtcaaagga aaagaccgcc ttggcattca gcctgtcccg   540

cagcacgata ttgcaatcca tctgcgccaa gctgaagaac gcggcatcaa ccttccggtc   600

actattgcgc tcggctgtga gccggtcatt acaacggcgg catcgactcc gcttctctat   660

gatcaatcag aatacgaaat ggcaggtgcg attcaaggcg aaccatatcg catcgtcaaa   720

tcaaagctgt ctgatcttga tgttccgtgg ggcgctgaag tggtgcttga aggtgagatt   780

attgccggag agcgcgaata tgaagggccg ttcggtgaat tcacaggcca ttattccggc 840

ggacgcagca tgccgattat caaaattaaa cgcgtctatc acagaaacaa tccgatcttt 900

gaacatttat acttaggcat gccttggaca gaatgcgatt acatgatcgg cattaacaca 960

tgcgtgccgc tttatcagca gttaaaagaa gcgtatccga acgaaattgt ggcagtgaac 1020

gccatgtaca cacacggttt aatcgcgatt gtttccacaa aaacccgcta tggcggattt 1080

gcgaaagcgg tcggcatgcg cgcactcaca acgccgcacg gactcggcta ctgcaaaatg 1140

gtcatagtcg ttgatgagga tgtcgatcca ttcaaccttc cgcaggtcat gtgggcgctt 1200

tcgaccaaaa tgcatccgaa acatgatgcg gtcatcattc cggacttatc tgtcctgccg 1260

cttgatccgg gatccaatcc atcaggaatc actcacaaaa tgattctcga cgccactaca 1320

ccggttgcgc cggaaacaag aggccattat tcacagccgc ttgattctcc gctaacaacg 1380

aaagaatggg aacaaaaact aatggactta atgaataaat aa 1422

<210> 4
<211> 473
<212> PRT
<213> Bacillus subtilis

<400> 4
Met Ala Tyr Gln Asp Phe Arg Glu Phe Leu Ala Ala Leu Glu Lys Glu
1               5                   10                  15

Gly Gln Leu Leu Thr Val Asn Glu Glu Val Lys Pro Glu Pro Asp Leu
            20                  25                  30

Gly Ala Ser Ala Arg Ala Ala Ser Asn Leu Gly Asp Lys Ser Pro Ala
        35                  40                  45

Leu Leu Phe Asn Asn Ile Tyr Gly Tyr His Asn Ala Arg Ile Ala Met
    50                  55                  60

Asn Val Ile Gly Ser Trp Pro Asn His Ala Met Met Leu Gly Met Pro
65                  70                  75                  80

Lys Asp Thr Pro Val Lys Glu Gln Phe Phe Glu Phe Ala Lys Arg Tyr
                85                  90                  95

Asp Gln Phe Pro Met Pro Val Lys Arg Glu Glu Thr Ala Pro Phe His
            100                 105                 110

Glu Asn Glu Ile Thr Glu Asp Ile Asn Leu Phe Asp Ile Leu Pro Leu
        115                 120                 125

Phe Arg Ile Asn Gln Gly Asp Gly Gly Tyr Tyr Leu Asp Lys Ala Cys
    130                 135                 140

Val Ile Ser Arg Asp Leu Glu Asp Pro Asp Asn Phe Gly Lys Gln Asn
145                 150                 155                 160

Val Gly Ile Tyr Arg Met Gln Val Lys Gly Lys Asp Arg Leu Gly Ile
                165                 170                 175

Gln Pro Val Pro Gln His Asp Ile Ala Ile His Leu Arg Gln Ala Glu

180                 185                 190

Glu Arg Gly Ile Asn Leu Pro Val Thr Ile Ala Leu Gly Cys Glu Pro
        195                 200                 205

Val Ile Thr Thr Ala Ala Ser Thr Pro Leu Leu Tyr Asp Gln Ser Glu
    210                 215                 220

Tyr Glu Met Ala Gly Ala Ile Gln Gly Glu Pro Tyr Arg Ile Val Lys
225                 230                 235                 240

Ser Lys Leu Ser Asp Leu Asp Val Pro Trp Gly Ala Glu Val Val Leu
                245                 250                 255

Glu Gly Glu Ile Ile Ala Gly Glu Arg Glu Tyr Glu Gly Pro Phe Gly
            260                 265                 270

Glu Phe Thr Gly His Tyr Ser Gly Gly Arg Ser Met Pro Ile Ile Lys
        275                 280                 285

Ile Lys Arg Val Tyr His Arg Asn Asn Pro Ile Phe Glu His Leu Tyr
    290                 295                 300

Leu Gly Met Pro Trp Thr Glu Cys Asp Tyr Met Ile Gly Ile Asn Thr
305                 310                 315                 320

Cys Val Pro Leu Tyr Gln Gln Leu Lys Glu Ala Tyr Pro Asn Glu Ile
                325                 330                 335

Val Ala Val Asn Ala Met Tyr Thr His Gly Leu Ile Ala Ile Val Ser
            340                 345                 350

Thr Lys Thr Arg Tyr Gly Gly Phe Ala Lys Ala Val Gly Met Arg Ala
        355                 360                 365

Leu Thr Thr Pro His Gly Leu Gly Tyr Cys Lys Met Val Ile Val Val
    370                 375                 380

Asp Glu Asp Val Asp Pro Phe Asn Leu Pro Gln Val Met Trp Ala Leu
385                 390                 395                 400

Ser Thr Lys Met His Pro Lys His Asp Ala Val Ile Ile Pro Asp Leu
                405                 410                 415

Ser Val Leu Pro Leu Asp Pro Gly Ser Asn Pro Ser Gly Ile Thr His
            420                 425                 430

Lys Met Ile Leu Asp Ala Thr Thr Pro Val Ala Pro Glu Thr Arg Gly
        435                 440                 445

His Tyr Ser Gln Pro Leu Asp Ser Pro Leu Thr Thr Lys Glu Trp Glu
    450                 455                 460

Gln Lys Leu Met Asp Leu Met Asn Lys
465                 470


<210>    5
<211>    228
<212>    DNA
<213>    Bacillus subtilis

<400>    5

atgcatacat gtcctcgatg cgactcaaaa aagggagaag tcatgagcaa atcgcctgta 60

gaaggcgcat gggaagttta tcagtgccaa acatgctttt ttacatggag atcctgtgaa 120

ccggaaagca ttacaaatcc cgaaaaatac aatccagcgt ttaaaattga tccaaaggaa 180

acagaaacag caattgaagt tccggcggtg ccggaacgaa aggcttga 228

<210> 6
<211> 75
<212> PRT
<213> Bacillus subtilis

<400> 6
Met His Thr Cys Pro Arg Cys Asp Ser Lys Lys Gly Glu Val Met Ser
1               5                   10                  15

Lys Ser Pro Val Glu Gly Ala Trp Glu Val Tyr Gln Cys Gln Thr Cys
            20                  25                  30

Phe Phe Thr Trp Arg Ser Cys Glu Pro Glu Ser Ile Thr Asn Pro Glu
        35                  40                  45

Lys Tyr Asn Pro Ala Phe Lys Ile Asp Pro Lys Glu Thr Glu Thr Ala
    50                  55                  60

Ile Glu Val Pro Ala Val Pro Glu Arg Lys Ala
65                  70                  75

<210> 7
<211> 1107
<212> DNA
<213> S.cerevisiae

<400> 7
atgaaaataa aaattttagt agatgaatat aatacgcaga aagaacaggc taaaaaagta 60

cgaaaagcaa cttcattata tttccgcatt catccttcaa ttatggccag caagactttg 120

agggttcttt ttctgggtcc caaaggtacg tattcccatc aagctgcatt acaacaattt 180

caatcaacat ctgatgttga gtacctccca gcagcctcta tcccccaatg ttttaaccaa 240

ttggagaacg acactagtat agattattca gtggtaccgt tggaaaattc caccaatgga 300

caagtagttt tttcctatga tctcttgcgt gataggatga tcaaaaaagc cctatcctta 360

cctgctccag cagatactaa tagaattaca ccagatatag aagttatagc ggagcaatat 420

gtacccatta cccattgtct aatcagccca atccaactac caaatggtat tgcatccctt 480

ggaaattttg aagaagtcat aatacactca catccgcaag tatggggcca ggttgaatgt 540

tacttaaggt ccatggcaga aaaatttccg caggtcacct ttataagatt ggattgttct 600

tccacatctg aatcagtgaa ccaatgcatt cggtcatcaa cggccgattg cgacaacatt 660

ctgcatttag ccattgctag tgaaacagct gcccaattgc ataaggcgta catcattgaa 720

cattcgataa atgataagct aggaaataca acaagatttt tagtattgaa gagaagggag 780

aacgcaggcg acaatgaagt agaagacact ggattactac gggttaacct actcaccttt 840

actactcgtc aagatgaccc tggttctttg gtagatgttt tgaacatact aaaaatccat 900

tcactcaaca tgtgttctat aaactctaga ccattccatt tggacgaaca tgatagaaac 960

tggcgatatt tatttttcat tgaatattac accgagaaga ataccccaaa gaataaagaa 1020

aaattctatg aagatatcag cgacaaaagt aaacagtggt gcctgtgggg tacattcccc 1080

agaaatgaga gatattatca caaataa 1107

<210> 8
<211> 368
<212> PRT
<213> S.cerevisiae

<400> 8
Met Lys Ile Lys Ile Leu Val Asp Glu Tyr Asn Thr Gln Lys Glu Gln
1               5                   10                  15

Ala Lys Lys Val Arg Lys Ala Thr Ser Leu Tyr Phe Arg Ile His Pro
            20                  25                  30

Ser Ile Met Ala Ser Lys Thr Leu Arg Val Leu Phe Leu Gly Pro Lys
        35                  40                  45

Gly Thr Tyr Ser His Gln Ala Ala Leu Gln Gln Phe Gln Ser Thr Ser
    50                  55                  60

Asp Val Glu Tyr Leu Pro Ala Ala Ser Ile Pro Gln Cys Phe Asn Gln
65                  70                  75                  80

Leu Glu Asn Asp Thr Ser Ile Asp Tyr Ser Val Val Pro Leu Glu Asn
                85                  90                  95

Ser Thr Asn Gly Gln Val Val Phe Ser Tyr Asp Leu Leu Arg Asp Arg
            100                 105                 110

Met Ile Lys Lys Ala Leu Ser Leu Pro Ala Pro Ala Asp Thr Asn Arg
        115                 120                 125

Ile Thr Pro Asp Ile Glu Val Ile Ala Glu Gln Tyr Val Pro Ile Thr
    130                 135                 140

His Cys Leu Ile Ser Pro Ile Gln Leu Pro Asn Gly Ile Ala Ser Leu
145                 150                 155                 160

Gly Asn Phe Glu Glu Val Ile Ile His Ser His Pro Gln Val Trp Gly
                165                 170                 175

Gln Val Glu Cys Tyr Leu Arg Ser Met Ala Glu Lys Phe Pro Gln Val
            180                 185                 190

Thr Phe Ile Arg Leu Asp Cys Ser Ser Thr Ser Glu Ser Val Asn Gln
        195                 200                 205

Cys Ile Arg Ser Ser Thr Ala Asp Cys Asp Asn Ile Leu His Leu Ala
    210                 215                 220

Ile Ala Ser Glu Thr Ala Ala Gln Leu His Lys Ala Tyr Ile Ile Glu
225                 230                 235                 240

His Ser Ile Asn Asp Lys Leu Gly Asn Thr Thr Arg Phe Leu Val Leu
                245                 250                 255

Lys Arg Arg Glu Asn Ala Gly Asp Asn Glu Val Glu Asp Thr Gly Leu
            260                 265                 270

Leu Arg Val Asn Leu Leu Thr Phe Thr Thr Arg Gln Asp Asp Pro Gly
        275                 280                 285

Ser Leu Val Asp Val Leu Asn Ile Leu Lys Ile His Ser Leu Asn Met
    290                 295                 300

Cys Ser Ile Asn Ser Arg Pro Phe His Leu Asp Glu His Asp Arg Asn
305                 310                 315                 320

Trp Arg Tyr Leu Phe Phe Ile Glu Tyr Tyr Thr Glu Lys Asn Thr Pro
                325                 330                 335

Lys Asn Lys Glu Lys Phe Tyr Glu Asp Ile Ser Asp Lys Ser Lys Gln
            340                 345                 350

Trp Cys Leu Trp Gly Thr Phe Pro Arg Asn Glu Arg Tyr Tyr His Lys
        355                 360                 365

<210>    9
<211>    1125
<212>    DNA
<213>    S.cerevisiae

<400>    9
atgtcactaa tggacaattg gaagactgat atggaaagtt acgatgaagg aggcctagtt    60

gctaatccga acttcgaggt tctggccact ttcaggtacg accctggttt tgcacgccag   120

tcagcgtcaa agaaagagat ctttgaaact ccagaccctc gattaggttt gagagacgaa   180

gatattaggc agcagataat taatgaggat tactcaagtt atttacgagt aagggaggtt   240

aattccggcg gtgaccttct cgaaaatatt cagcatcctg atgcttggaa gcatgattgc   300

aagaccattg tgtgccagcg tgtagaagat atgctacaag tcatttatga acgatttttt   360

ttattagatg aacaatacca aagaataaga atagcattat catactttaa aattgacttc   420

agcacgtctc tgaatgattt attgaagtta ttggttgaaa acttgattaa ttgtaaagaa   480

ggaaattcag agtatcacga aaaaattcaa aaaatgatca acgaaaggca atgctataaa   540

atgcgggtac ttgtctctaa gacaggagat atacgaattg aggcaattcc aatgcctatg   600

gagcctatcc taaaattaac aaccgattat gacagtgttt ccacatactt catcaaaacg   660

atgctcaatg gatttttaat tgatagcaca ataaattggg atgttgttgt ttcatctgaa   720

ccattgaacg catcagcttt caccagtttt aaaaccactt caagagatca ttacgctagg   780

gcgagagttc gcatgcaaac tgctataaat aacttaagag gttcagaacc tacttcttct   840

gtctcgcaat gcgaaatttt attttccaac aaatctggcc tgctgatgga aggttcaata   900

acaaacgtgg ctgtaattca aaaagatcct aacggttcta aaaagtatgt gacaccaaga 960

ttagcaactg gatgtttgtg cggaacaatg cgtcattatt tattgcggct cggccttatt 1020

gaagagggag atatagatat aggaagcctt accgttggca acgaagtttt gcttttcaat 1080

ggcgtcatgg gatgcataaa gggaacagtg aagacaaaat attga 1125

<210> 10
<211> 374
<212> PRT
<213> S.cerevisiae

<400> 10
Met Ser Leu Met Asp Asn Trp Lys Thr Asp Met Glu Ser Tyr Asp Glu
1               5                   10                  15

Gly Gly Leu Val Ala Asn Pro Asn Phe Glu Val Leu Ala Thr Phe Arg
            20                  25                  30

Tyr Asp Pro Gly Phe Ala Arg Gln Ser Ala Ser Lys Lys Glu Ile Phe
        35                  40                  45

Glu Thr Pro Asp Pro Arg Leu Gly Leu Arg Asp Glu Asp Ile Arg Gln
    50                  55                  60

Gln Ile Ile Asn Glu Asp Tyr Ser Ser Tyr Leu Arg Val Arg Glu Val
65                  70                  75                  80

Asn Ser Gly Gly Asp Leu Leu Glu Asn Ile Gln His Pro Asp Ala Trp
                85                  90                  95

Lys His Asp Cys Lys Thr Ile Val Cys Gln Arg Val Glu Asp Met Leu
            100                 105                 110

Gln Val Ile Tyr Glu Arg Phe Phe Leu Leu Asp Glu Gln Tyr Gln Arg
        115                 120                 125

Ile Arg Ile Ala Leu Ser Tyr Phe Lys Ile Asp Phe Ser Thr Ser Leu
    130                 135                 140

Asn Asp Leu Leu Lys Leu Leu Val Glu Asn Leu Ile Asn Cys Lys Glu
145                 150                 155                 160

Gly Asn Ser Glu Tyr His Glu Lys Ile Gln Lys Met Ile Asn Glu Arg
                165                 170                 175

Gln Cys Tyr Lys Met Arg Val Leu Val Ser Lys Thr Gly Asp Ile Arg
            180                 185                 190

Ile Glu Ala Ile Pro Met Pro Met Glu Pro Ile Leu Lys Leu Thr Thr
        195                 200                 205

Asp Tyr Asp Ser Val Ser Thr Tyr Phe Ile Lys Thr Met Leu Asn Gly
    210                 215                 220

Phe Leu Ile Asp Ser Thr Ile Asn Trp Asp Val Val Val Ser Ser Glu
225                 230                 235                 240

Pro Leu Asn Ala Ser Ala Phe Thr Ser Phe Lys Thr Thr Ser Arg Asp
                245                 250                 255

His Tyr Ala Arg Ala Arg Val Arg Met Gln Thr Ala Ile Asn Asn Leu
            260                 265                 270

Arg Gly Ser Glu Pro Thr Ser Ser Val Ser Gln Cys Glu Ile Leu Phe
        275                 280                 285

Ser Asn Lys Ser Gly Leu Leu Met Glu Gly Ser Ile Thr Asn Val Ala
    290                 295                 300

Val Ile Gln Lys Asp Pro Asn Gly Ser Lys Lys Tyr Val Thr Pro Arg
305                 310                 315                 320

Leu Ala Thr Gly Cys Leu Cys Gly Thr Met Arg His Tyr Leu Leu Arg
                325                 330                 335

Leu Gly Leu Ile Glu Glu Gly Asp Ile Asp Ile Gly Ser Leu Thr Val
            340                 345                 350

Gly Asn Glu Val Leu Leu Phe Asn Gly Val Met Gly Cys Ile Lys Gly
        355                 360                 365

Thr Val Lys Thr Lys Tyr
    370

<210>    11
<211>    1560
<212>    DNA
<213>    S.cerevisiae

<400>    11
atgttcagta gatctacgct ctgcttaaag acgtctgcat cctccattgg gagacttcaa    60

ttgagatatt tctcacacct tcctatgaca gtgcctatca agctgcccaa tgggttggaa   120

tatgagcaac caacggggtt gttcatcaac aacaagtttg ttccttctaa acagaacaag   180

accttcgaag tcattaaccc ttccacggaa gaagaaatat gtcatattta tgaaggtaga   240

gaggacgatg tggaagaggc cgtgcaggcc gccgaccgtg ccttctctaa tgggtcttgg   300

aacggtatcg accctattga caggggtaag gctttgtaca ggttagccga attaattgaa   360

caggacaagg atgtcattgc ttccatcgag actttggata acggtaaagc tatctcttcc   420

tcgagaggag atgttgattt agtcatcaac tatttgaaat cttctgctgg ctttgctgat   480

aaaattgatg gtagaatgat tgatactggt agaacccatt tttcttacac taagagacag   540

cctttgggtg tttgtgggca gattattcct tggaatttcc cactgttgat gtgggcctgg   600

aagattgccc ctgctttggt caccggtaac accgtcgtgt tgaagactgc cgaatccacc   660

ccattgtccg ctttgtatgt gtctaaatac atcccacagg cgggtattcc acctggtgtg   720

atcaacattg tatccgggtt tggtaagatt gtgggtgagg ccattacaaa ccatccaaaa   780

atcaaaaagg ttgccttcac agggtccacg gctacgggta gacacattta ccagtccgca   840

gccgcaggct tgaaaaaagt gactttggag ctgggtggta aatcaccaaa cattgtcttc   900

gcggacgccg agttgaaaaa agccgtgcaa aacattatcc ttggtatcta ctacaattct   960

ggtgaggtct gttgtgcggg ttcaagggtg tatgttgaag aatctattta cgacaaattc 1020

attgaagagt tcaaagccgc ttctgaatcc atcaaggtgg gcgacccatt cgatgaatct 1080

actttccaag gtgcacaaac ctctcaaatg caactaaaca aaatcttgaa atacgttgac 1140

attggtaaga atgaaggtgc tactttgatt accggtggtg aaagattagg tagcaagggt 1200

tacttcatta agccaactgt ctttggtgac gttaaggaag acatgagaat tgtcaaagag 1260

gaaatctttg gccctgttgt cactgtaacc aaattcaaat ctgccgacga agtcattaac 1320

atggcgaacg attctgaata cgggttggct gctggtattc acacctctaa tattaatacc 1380

gccttaaaag tggctgatag agttaatgcg ggtacggtct ggataaacac ttataacgat 1440

ttccaccacg cagttccttt cggtgggttc aatgcatctg gtttgggcag ggaaatgtct 1500

gttgatgctt tacaaaacta cttgcaagtt aaagcggtcc gtgccaaatt ggacgagtaa 1560

1560

<210> 12
<211> 519
<212> PRT
<213> S.cerevisiae

<400> 12
Met Phe Ser Arg Ser Thr Leu Cys Leu Lys Thr Ser Ala Ser Ser Ile
1               5                   10                  15

Gly Arg Leu Gln Leu Arg Tyr Phe Ser His Leu Pro Met Thr Val Pro
            20                  25                  30

Ile Lys Leu Pro Asn Gly Leu Glu Tyr Glu Gln Pro Thr Gly Leu Phe
        35                  40                  45

Ile Asn Asn Lys Phe Val Pro Ser Lys Gln Asn Lys Thr Phe Glu Val
    50                  55                  60

Ile Asn Pro Ser Thr Glu Glu Glu Ile Cys His Ile Tyr Glu Gly Arg
65                  70                  75                  80

Glu Asp Asp Val Glu Glu Ala Val Gln Ala Ala Asp Arg Ala Phe Ser
                85                  90                  95

Asn Gly Ser Trp Asn Gly Ile Asp Pro Ile Asp Arg Gly Lys Ala Leu
            100                 105                 110

Tyr Arg Leu Ala Glu Leu Ile Glu Gln Asp Lys Asp Val Ile Ala Ser
        115                 120                 125

Ile Glu Thr Leu Asp Asn Gly Lys Ala Ile Ser Ser Ser Arg Gly Asp
    130                 135                 140

Val Asp Leu Val Ile Asn Tyr Leu Lys Ser Ser Ala Gly Phe Ala Asp
145                 150                 155                 160

Lys Ile Asp Gly Arg Met Ile Asp Thr Gly Arg Thr His Phe Ser Tyr
                165                 170                 175

Thr Lys Arg Gln Pro Leu Gly Val Cys Gly Gln Ile Ile Pro Trp Asn
            180                 185                 190

Phe Pro Leu Leu Met Trp Ala Trp Lys Ile Ala Pro Ala Leu Val Thr
        195                 200                 205

Gly Asn Thr Val Val Leu Lys Thr Ala Glu Ser Thr Pro Leu Ser Ala
    210                 215                 220

Leu Tyr Val Ser Lys Tyr Ile Pro Gln Ala Gly Ile Pro Pro Gly Val
225                 230                 235                 240

Ile Asn Ile Val Ser Gly Phe Gly Lys Ile Val Gly Glu Ala Ile Thr
                245                 250                 255

Asn His Pro Lys Ile Lys Lys Val Ala Phe Thr Gly Ser Thr Ala Thr
            260                 265                 270

Gly Arg His Ile Tyr Gln Ser Ala Ala Ala Gly Leu Lys Lys Val Thr
        275                 280                 285

Leu Glu Leu Gly Gly Lys Ser Pro Asn Ile Val Phe Ala Asp Ala Glu
    290                 295                 300

Leu Lys Lys Ala Val Gln Asn Ile Ile Leu Gly Ile Tyr Tyr Asn Ser
305                 310                 315                 320

Gly Glu Val Cys Cys Ala Gly Ser Arg Val Tyr Val Glu Glu Ser Ile
                325                 330                 335

Tyr Asp Lys Phe Ile Glu Glu Phe Lys Ala Ala Ser Glu Ser Ile Lys
            340                 345                 350

Val Gly Asp Pro Phe Asp Glu Ser Thr Phe Gln Gly Ala Gln Thr Ser
        355                 360                 365

Gln Met Gln Leu Asn Lys Ile Leu Lys Tyr Val Asp Ile Gly Lys Asn
    370                 375                 380

Glu Gly Ala Thr Leu Ile Thr Gly Gly Glu Arg Leu Gly Ser Lys Gly
385                 390                 395                 400

Tyr Phe Ile Lys Pro Thr Val Phe Gly Asp Val Lys Glu Asp Met Arg
                405                 410                 415

Ile Val Lys Glu Glu Ile Phe Gly Pro Val Val Thr Val Thr Lys Phe
            420                 425                 430

Lys Ser Ala Asp Glu Val Ile Asn Met Ala Asn Asp Ser Glu Tyr Gly
        435                 440                 445

Leu Ala Ala Gly Ile His Thr Ser Asn Ile Asn Thr Ala Leu Lys Val
    450                 455                 460

Ala Asp Arg Val Asn Ala Gly Thr Val Trp Ile Asn Thr Tyr Asn Asp
465                 470                 475                 480

Phe His His Ala Val Pro Phe Gly Gly Phe Asn Ala Ser Gly Leu Gly
                485                 490                 495

Arg Glu Met Ser Val Asp Ala Leu Gln Asn Tyr Leu Gln Val Lys Ala
            500                 505                 510

Val Arg Ala Lys Leu Asp Glu
        515

<210>    13
<211>    29
<212>    DNA
<213>    Artificial Sequence

<220>
<223>    forward primer 1

<400>    13
aaacatatga aaataaaaat tttagtaga                                          29

<210>    14
<211>    29
<212>    DNA
<213>    Artificial Sequence

<220>
<223>    backward primer 1

<400>    14
aaactcgagt ttgtgataat atctctcat                                          29

<210>    15
<211>    29
<212>    DNA
<213>    Artificial Sequence

<220>
<223>    forward primer 2

<400>    15
aaacatatga aagcagaatt caagcgtaa                                          29

<210>    16
<211>    29
<212>    DNA
<213>    Artificial Sequence

<220>
<223>    backward primer 2

<400>    16
aaactcgaga gcctttcgtt ccggcaccg                                          29

<210>    17
<211>    29
<212>    DNA
<213>    Artificial Sequence

<220>
<223>    forward primer 3

<400>    17
aaacatatgt cactaatgga caattggaa                                          29

<210>    18
<211>    29
<212>    DNA
<213>    Artificial Sequence

<220>
<223>    backward primer 3

<400>    18
aaactcgaga tattttgtct tcactgttc                                          29